# EUROPEAN PATENT APPLICATION

(11) **EP 2 399 625 A1**
(43) Date of publication of application: **28.12.2011**
(21) Application number: 10743674.3
(22) Date of filing: 10.02.2010
(51) Int. Cl.: A61M 5/158, A61M 5/32

(54) **MEDICAL NEEDLE DEVICE**

(30) Priority: 19.02.2009 JP 2009036614; 09.02.2010 JP 2010026541
(71) Applicant: JMS Co., Ltd., Hiroshima-shi Hiroshima 730-8652 (JP)
(72) Inventor: NAKANISHI, Takahide, Miyoshi-shi, Hiroshima 728-0016 (JP); SANADA, Eiji, Miyoshi-shi, Hiroshima 728-0016 (JP)
(74) Representative: Winkler, Andreas Fritz Ernst
(86) International application number: PCT/JP2010/051952
(87) International publication number: WO 2010/095547

(57) **Abstract**

A medical needle device 71 comprises: a hub 10 having an injection needle 11 and a tube 40 respectively fixed at a top end and a rear end thereof; a substantially cylindrical sheath 80 which has a wing 51 mounted thereon and supports the hub 10 in an interior side thereof; and a protector 82 fit on the sheath 80 where the sheath 80 is slidable back and forth from a puncturing position to a storing position, wherein: the protector 82 comprises: a circular front part 82a; a circular rear part 82b; and connecting parts 86 connecting the front part 82a and the rear part 82b, which have spaces for protruding a pair of wings 51, 52 on the sheath 80 therefrom for enabling slide of the sheath 80; and an outer peripheral surface of the sheath 80 and a front side and a rear side of the protector 82 form a position controlling mechanism 81, 85 for positioning and fixing the sheath 80 relative to the protector 82 on the puncturing position and the storing position.

## Description

### TECHNICAL FIELD

The present invention relates to medical needle devices with wings to be fastened on the patient's skin with adhesive tape, which are used for measures such as infusions or blood transfusions, or extracorporeal blood circulation.

### BACKGROUND OF THE INVENTION

For measures such as blood dialysis therapy, infusions or blood transfusions, or extracorporeal blood circulation, winged medical needle devices fastened on the patient's skin with adhesive tape are widely used.

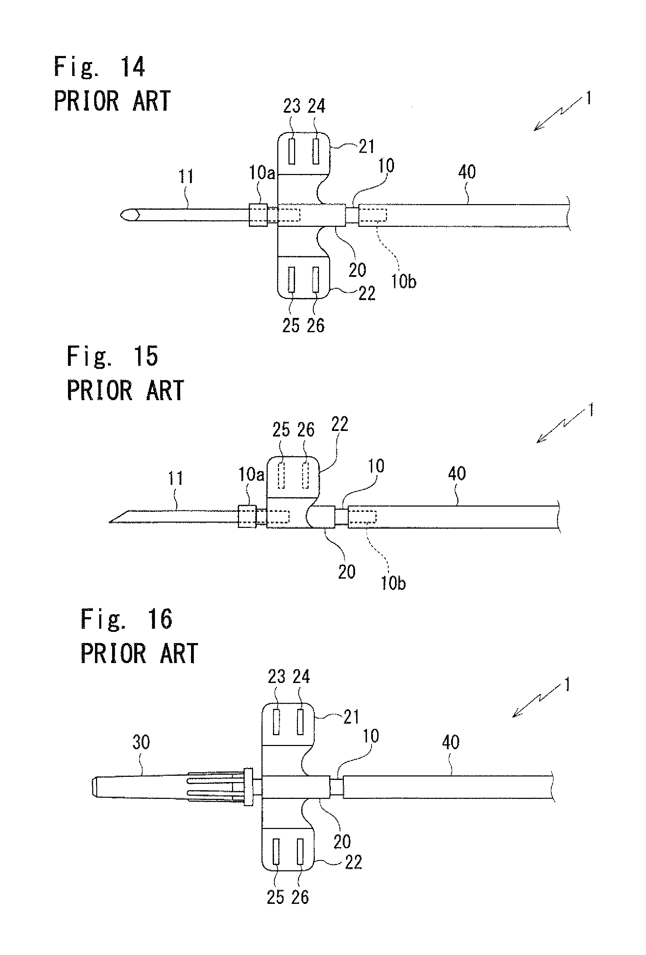

Such medical needle devices as shown in Fig. 14 comprise a hub 10 having an injection needle 11 fixed at the top end 10a thereof and a tube 40 connected to a rear end 10b thereof, and a substantially cylindrical sheath 20 which is fit on the hub 10, having a pair of wings 21, 22 respectively mounted on both right and left side surfaces thereof and supporting the hub 10 in an interior side thereof. The wings 21, 22 have convexes 23, 26 and grooves 24, 25 formed thereon and when the wings 21 and 22 are overlapped as shown in Fig. 15, the convex 23 engages with the groove 25 whereas the convex 26 engages with the groove 24.

The above-structured medical needle devices are used as follows; the injection needle 11 is set on a blood vessel of a patient and then the blood vessel is secured; a pair of the wings 21, 22 are spread apart so as to come into contact with the patient's skin and are fastened on the patient's skin with adhesive tape (not shown); after medical treatment, the injection needle 11 is removed from the patient's skin and the medical needle device is disposed with a protective cap 30 capped on the injection needle 11 as shown in Fig. 16.

The above-structured medical needle devices, however, have caused a problem that the users may erroneously prick fingers with the injection needle after use when holding the used medical needle device in one hand and capping the injection needle 11 with the cap 30 with the other hand, which causes infection of HIV or hepatitis.

In this connection, there have been proposed the medical needle devices shown in Fig. 17 to Fig. 22 (see, for example, the Japanese examined Patent Publication No. H06-7861).

The Japanese examined Patent Publication No. H06-7861 discloses a medical needle device comprising: a hub 10 having the injection needle 11 fixed at the top end 10a thereof and the tube 40 connected to the rear end 10b thereof, and a protector 50 slidably fit on the hub 10 back and forth from a puncturing position where the injection needle 11 is protruded to a storing position where the injection needle 11 is stored and having a pair of wings 51, 52 respectively mounted on both right and left side surfaces thereof.

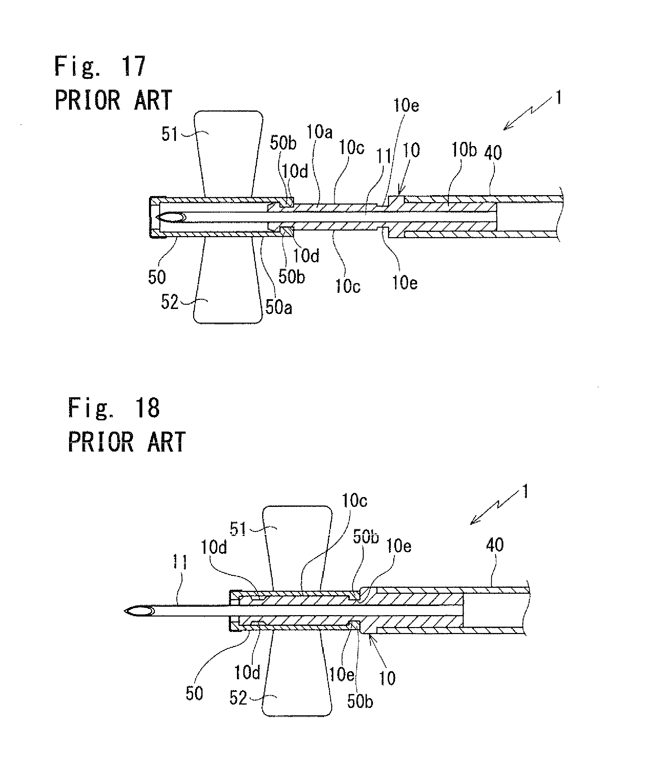

The hub 10 has two shallow ditches 10c formed on an outer periphery of the top end 10a thereof at intervals of 180 degrees in a direction of circumferential surface. The ditches 10c have concaves 10d, 10e formed on a top end side and a bottom end side thereof, which are deeper than the ditches 10c by one step. The protector 50 has two retaining protrusions 50b protrudingly provided on an internal circumference on a back end 50a thereof. The retaining protrusions 50b are fit with the concaves 10d on the top end 10a of the hub 10 before use of the medical needle device (Fig. 17) and are moved through the ditches 10c of the hub 10 and fit with the concaves 10e on the rear end 10b side of the hub 10 (Fig. 18) at the time of the use of the medical needle device. After the use of the medical needle device, the retaining protrusions 50b are moved through the ditches 10c of the hub 10 again and are fit with the concaves 10d on the top end 10a of the hub 10 again. The injection needle 11 may be capped with the cap (not shown) before the use of the medical needle device.

The Japanese examined Patent Publication No. H06-7861 also discloses a medical needle device shown in Figs. 19 to 22, of which wings 51, 52 are integrally formed with a hub 10 while a protector 60 is separately provided.

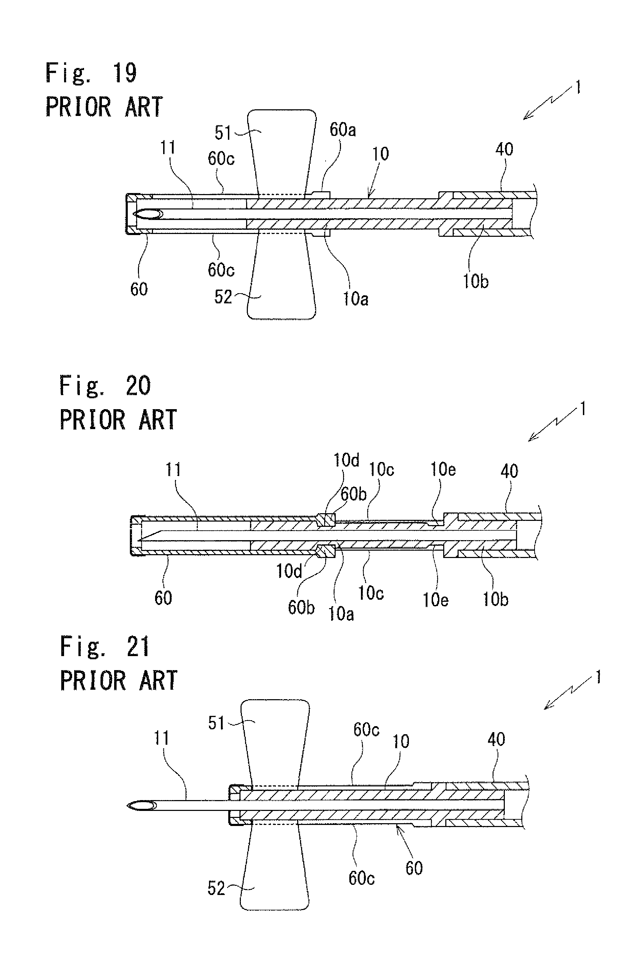

The protector 60 has: slits 60c which extend toward a rear end 60a side and are open on the rear end 60a; and retaining protrusions 60b protrudingly provided on an inner surface of the rear end 60a of semicylindrical parts which are two parts of the protector 60 split by the slits 60c. The protector 60 slidably covers the hub 10 back and forth from a puncturing position to a storing position with the wings 51, 52 protruding from the slits 60c. The puncturing position is a position where the injection needle 11 is protruded (Fig. 21) and the storing position is a position where the injection needle 11 is stored (Figs. 19, 20) with the wings 51, 52 protrude from the slits 60c.

According to the invention shown in Figs. 17 and 18, however, the protector 50 and the wings 51, 52 are integrally formed, which necessitates formation of the protector 50 and the wings 51, 52 by the same material. In another word, since the wings 51, 52 are required to be flexible, the protector 50 also has to be made of soft material with the flexibility. Accordingly, while the protector 50 caps the injection needle 11, there remains possibility that the injection needle 11 goes through a side surface of the protector 50 and pricks fingers of doctors or the like, thereby remaining safety problems.

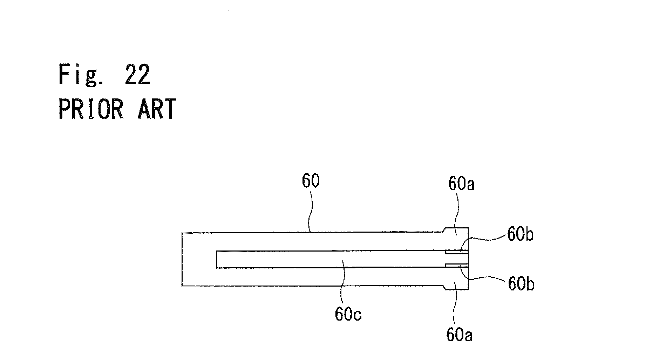

According to the invention shown in Figs. 19 to 22, since the slits 60c on the protector 60 are open on the rear end 60a, the protector 60 falls into an outward relief state by the retaining protrusions 60b, thereby weakening fitting force of the protector 60 with the hub 10. Accordingly, the protector 60 can come off from the hub 10, thereby lacking in stability.

The inventions shown in Figs. 17 through 22 also have a common problem that the retaining protrusions 50b, 60b of the protectors 50, 60 are so-called undercuts so that formation thereof is relatively complicated.

Therefore, an object of the present invention is to provide medical needle devices which are capable of preventing the injection needles from pricking the fingers and easily formed.

### DISCLOSURE OF THE INVENTION

In order to achieve the above-mentioned object, a first aspect of the invention provides a medical needle device (71) comprising: a hub (10) having an injection needle (11) and a tube (40) respectively fixed at a top end and a rear end thereof; a cylindrical sheath (80, 92) which is fit on the hub (10), having a pair of wings (51, 52) mounted on both right and left side surfaces thereof and supporting the hub (10) in an interior side thereof and a protector (82, 89, 94, 99) fit on the sheath (80, 92), where said sheath (80, 92) together with the hub (10) is slidable back and forth from a puncturing position where the injection needle (11) is protruded to a storing position where the injection needle (11) is stored, wherein:
said protector (82, 89, 94, 99) comprises: a circular front part (82a, 89a, 94a, 99a) which said injection needle (11) goes through; a circular rear part (82b, 89b, 94b, 99b) which said tube (40) goes through; and connecting parts (86, 90, 95) connecting said front part (82a, 89a, 94a, 99a) and said rear part (82b, 89b, 94b, 99b), which have spaces for protruding said pair of wings (51, 52) of said sheath (80, 92) therefrom for enabling slide of said sheath (80, 92); said protector (82, 89, 94, 99) is made of harder material than that of said sheath (80, 92); and an outer peripheral surface of said sheath (80, 92) and a front side and a rear side of said protector (82, 89, 94, 99) form a position controlling mechanism for positioning and fixing said sheath (80, 92) relative to said protector (82, 89, 94, 99) on said puncturing position and said storing position.

A second aspect of the invention provides a medical needle device (71) according to the first aspect of the invention, wherein: said protector (82) comprises two half-split shape parts (83, 84) for an upper side and a lower side, covers said sheath (80) in such a manner as to fasten said sheath (80) from the upper side and the lower side and has the space between said half-split shape parts (83, 84) for enabling the slide of said sheath (80).

A third aspect of the invention provides a medical needle device (71) according to the second aspect of the invention, wherein: said position controlling mechanism comprises protrusions (81) formed on an upper part of the outer peripheral surface of said sheath (80) and anchoring holes (85) formed on the front side and the rear side of said protector (82), which fit with said protrusions (81); and said half-split shape part (83) for the upper side of said protector (82) has a guide ditch (87) formed between said anchoring holes (85) on the front side and rear side thereof for guiding said protrusions (81) back and forth at a time of said slide.

A fourth aspect of the invention provides a medical needle device (71) according to the second or the third aspect of the invention, wherein: both of said half-split shape parts (83, 84) for the upper side and the lower side have same shapes.

A fifth aspect of the invention provides a medical needle device (71) according to the first aspect of the invention, wherein: said connecting parts (90) of the protector (89) only connect an upper part of said front part (89a) and an upper part of said rear part (89b); said position controlling mechanism comprises the protrusion (81) formed on the upper part of the outer peripheral surface of said sheath (80) and the anchoring holes (85) formed on the front side and the rear side of the connecting parts (90) of said protector (89), which fit with said protrusion (81); and said connecting parts (90) have the guide ditch (87) formed between said anchoring holes (85) on the front side and the rear side thereof for guiding said protrusion (81) back and forth at the time of said slide.

A sixth aspect of the invention provides a medical needle device (71) according to the first aspect of the invention, wherein: said connecting parts (95) of said protector (94) comprise two parts that connect both right and left side surfaces of said front part (94a) and both right and left side surfaces of said rear part (94b); said position controlling mechanism comprises two protrusions (93) respectively formed on the right and left side surfaces of the outer peripheral surface of said sheath (92) and the anchoring holes (97) formed on the front side and the rear side of the two connecting parts (95) of said protector (94), which fit with said two protrusions (93); and said two connecting parts (95) respectively have the guide ditches (98) formed between said anchoring holes (97) on the front side and the rear side thereof for guiding said protrusions (93) back and forth at the time of said slide.

A seventh aspect of the invention provides a medical needle device (71) comprising: a hub (10) having an injection needle (11) and a tube (40) respectively fixed at a top and and a rear end thereof a cylindrical sheath (92) which is fit on the hub (10), having a pair of wings (51, 52) mounted on both right and left side surfaces thereof and supporting the hub (10) in an interior side thereof and a protector (99) fit on the sheath (92), where said sheath (92) together with the hub (10) is slidable back and forth from a puncturing position where the injection needle (11) is protruded to a storing position where the injection needle (11) is stored, wherein:
said protector (99) comprises: a circular front part (99a) which said injection needle (11) goes through; a half-split rear part (99b) of which lower part is cut out for fitting said tube (40) therein; and two connecting parts (95) which connect both right and left side surfaces of said front part (99a) and both right and left side surfaces of said rear part (99b), where said pair of wings (51, 52) on said sheath (92) protrude from lower parts of said connecting parts (95) for enabling slid of said sheath (92); said protector (99) is made of harder material than said sheath (92); two protrusions (93) formed both on right and left side surfaces of an outer peripheral surface of said sheath (92) and anchoring holes (97) formed on the front side and the rear side of the two connecting parts (95) of said protector (99), which fit with said protrusions (93) form a position controlling mechanism for positioning and fixing said sheath (92) relative to said protector (99) on said puncturing position and said storing position; and said two connecting parts (95) have guide ditches (98) formed between said anchoring holes (97) on the front side and the rear side thereof for guiding said protrusions (93) back and forth at a time of said slide.

A eighth aspect of the invention provides a medical needle device (71) according to any one of the first to the fourth aspects of the invention, wherein: said protector (82) includes covers (101) for narrowing spaces (200) between upper parts of said pair of wings (51, 52) on said sheath (92) and parts of said protector (82) facing said upper parts of a pair of said wings (51, 52) on said puncturing position and said storing position.

A ninth aspect of the invention provides a medical needle device (71) according to any one of the second to the fourth aspects of the invention, wherein: said protector (82) has inclined projections (102, 103) formed inside on the rear side of one (84) of two half-split shape parts (83, 84) for the upper side and the lower side, which incline said sheath (80) on said storing position so that the top end of said injection needle (11) abuts an inside on the front side of said protector (82).

A tenth aspect of the invention provides a medical needle device (71) according to any one of the second to the fourth aspects of the invention, wherein: an external shape of said sheath (80) is sectorial or inverted trapezoidal in cross section, tightening the lower part thereof to narrow gradually from the upper part; and an internal shape of a part of the half-split shape part for the upper side (83) of said protector (82) to be capped on said sheath (80) is sectorial or inverted trapezoidal in cross section, corresponding to a cross sectional shape of said sheath (80).

It is to be noted that the word "circular" in the present invention means a shape that is spatially closed and does not include a roughly circular shape of which a part is cut out as the letter "C". The word "circular" also includes any circular shape that is closed in addition to completely circular shape.

Symbols in parentheses show constituents or items corresponding to DRAWINGS and BEST MODE FOR CARRYING OUT THE INVENTION.

According to the medical needle device according to the first aspect of the present invention, the protector comprises: the circular front part which the injection needle goes through; the circular rear part which the tube goes through; and the position controlling mechanism for positioning and fixing the sheath relative to the protector on the puncturing position where the injection needle is protruded and the storing position where the injection needle is stored so that the injection needle hardly protrudes at the time of disposal of the medical needle device after the injection needle is fixed on the storing position, thereby enhancing safety. In another word, since the front part and the rear part of the protector are closed, deformation quantity of the protector is restricted while positioning and fixing the sheath on the puncturing position and the storing position, thereby enhancing fitting force. Accordingly, once the sheath is positioned and fixed on the storing position, the injection needle hardly protrudes again, thereby enhancing safety.

In addition, the rear part of the protector is circularly closed so that the wings hit the rear part of the protector when the sheath is moved backward relative to the protector and the sheath does not come off the protector. Accordingly, the injection needle is hardly exposed and the medical needle device can be safely disposed.

Since the protector is made of harder material than the sheath, even in case the top end of the injection needle slants and abuts the side surface of the protector on the storing position, the injection needle does not break through the side surface of the protector, thereby enhancing safety.

According to the medical needle device according to the second aspect of the present invention, in addition to the effect of the invention claimed in claim 1, the protector comprises two half-split shape parts for the upper side and the lower side so that the protector is simply molded. In another word, formation of the protector by two members prevents formation of so-called undercut on molds, thereby simplifying molding.

In addition, the two half-split shape parts fasten and cover the sheath from the upper side and the lower side so that the medical needle device is simply assembled.

According to the medical needle device according to the third aspect of the present invention, in addition to the effect of the invention claimed in claim 1 or 2, since the position controlling mechanism comprises the protrusions formed on the upper part of the outer peripheral surface of the sheath and the anchoring holes formed on the front side and the rear side of the protector, which fit with the protrusions, the sheath is positioned and fixed relative to the protector without fail on the puncturing position and the storing position. Accordingly, the structure prevents the injection needle from going into or protruding from the protector unexpectedly, thereby enhancing safety of the medical needle device.

In addition, the guide ditch for guiding the protrusions back and forth at the time of the slide prevents the sheath from rotating relative to an axis in which the protector extends even in case force is applied on the sheath or the wings in a rotational direction at the time of slide. In another word, since the protrusions are inside the guide ditches at the time of slide, the sheath does not rotate.

Further, the protrusions are moved from the anchoring hole to the guide ditch and from the guide ditch to the anchoring hole for position controlling. Accordingly, progress in each stage of operation can be recognized by sense of touch between the protrusions and the protector, which arises when moving the protrusions from the anchoring hole to the guide ditch and from the guide ditch to the anchoring hole. As a result, fitting state between the sheath and the protector can be recognized tactually as well as visually as in the conventional manner.

According to the medical needle device according to the fourth aspect of the present invention, in addition to the effect of the invention claimed in claim 2 or 3, both of the half-split shape parts for the upper side and the lower side have the same shapes, which necessitates one type of mold for molding the protectors. Accordingly, the medical needle device is cost effective and keeping parts before assembly becomes easier.

According to the medical needle device according to the fifth aspect of the present invention, in addition to the effect of the invention claimed in claim 1, the connecting parts of the protector only connect the upper part of the front part and the upper part of the rear part, and the connecting parts have the anchoring holes formed on the front side and the rear side thereof. Accordingly, although the protector comprises a single part, the protector does not cause undercut and is relatively easily molded. In addition, the protector which comprises the single part is cost effective.

According to the medical needle device according to the sixth aspect of the present invention, the connecting parts of the protector comprise two parts that connect both the right and left side surfaces of the front part and both the right and left side surfaces of the rear part; the position controlling mechanism comprises two protrusions respectively formed on the right and left side surfaces of the outer peripheral surface of the sheath and the anchoring holes formed on the front side and the rear side of the two connecting parts of the protector, which fit with the two protrusions; and the two connecting parts respectively have the guide ditches formed between the anchoring holes on the front side and the rear side thereof for guiding the protrusions back and forth at the time of the slide. Therefore, the medical needle device according to the sixth aspect of the present invention enables to visually confirm fitting state of the hub with the tube in addition to the effect of the invention according to the first aspect of the present invention.

Further, the protector which comprises the single part is cost effective.

According to the medical needle device according to the seventh aspect of the present invention, the protector comprises the circular front part which the injection needle goes through and the half-split rear part of which lower part is cut out for fitting the tube therein, and has the position controlling mechanism for positioning and fixing the sheath relative to the protector on the puncturing position where the injection needle is protruded and the storing position where the injection needle is stored. Accordingly, the injection needle hardly protrudes at the time of disposal of the medical needle device after the injection needle is fixed at the storing position, thereby enhancing safety, In another word, since the protector only has the lower part of the rear part thereof cut out, deformation quantity of the protector when positioning and fixing the sheath on the storing position is restricted and the fitting force is enhanced compared with a case in which both the upper and lower parts of a circumference are cut out. Accordingly, once the sheath is positioned and fixed on the storing position, the injection needle hardly protrudes again, thereby enhancing safety.

In addition, since the protector is made of harder material than the sheath, the top end of the injection needle does not break through the side surface of the protector thereby enhancing safety.

Further, the protector comprises half-split rear part of which lower part is cut out and two connecting parts which connect both right and left side surfaces of the front part and both right and left side surfaces of the rear part. Therefore, after the injection needle goes through the front part, a combined body comprising: the injection needle; the hub having the tube connected to the rear end thereof; and the sheath is fit with the protector. Accordingly, the assembly of the medical needle device is simplified.

Furthermore, the position controlling mechanism comprises the two protrusions formed both on the right and left side surfaces of the outer peripheral surface of the sheath and the anchoring holes formed on the front side and the rear side of the two connecting parts of the protector, which fit with the protrusions. Such a structure enables positioning and fixing the sheath relative to the protector without fail on the puncturing position and the storing position. Accordingly, the structure prevents the injection needle from going into or protruding from the protector unexpectedly, thereby enhancing safety of the medical needle device.

In addition, the guide ditches for guiding the protrusions back and forth at the time of the slide prevent the sheath from rotating even in case force is applied on the sheath or the wings in a rotational direction at the time of slide. In another word, since the protrusions are inside the guide ditches at the time of slide, the sheath does not rotate. Also, no part of the medical needle device obstructs the protrusions at the time of slide so that an excellent operability is attained.

Further, the protrusions are moved from the anchoring holes to the guide ditch and from the guide ditch to the anchoring holes for position controlling. Accordingly, progress in each stage of operation can be recognized by sense of touch between the protrusions and the protector, which arises when moving the protrusions from the anchoring holes to the guide ditch and from the guide ditch to the anchoring holes. As a result, fitting state between the sheath and the protector can be recognized tactually as well as visually as in the conventional manner.

In addition, the protector which comprises the single part is cost effective.

According to the medical needle device according to the eighth aspect of the present invention, the protector includes the covers for narrowing the spaces between the upper parts of the pair of wings on the sheath and the protector. Accordingly, even in case strong force is applied on the sheath or the wings in the rotational direction, which is so strong as to release the positioning and fixing by the position controlling mechanism, the wings immediately come into contact with the covers so that further rotation is prevented. The shorter the space between the upper parts of the wings and the protector is within a range in which the sheath is slidable, the smaller the movement in the rotational direction is.

In addition, formation of the covers to an extent that hide the top end of the injection needle leads to a sense of security to operators.

According to the medical needle device according to the ninth aspect of the present invention, the protector has inclined projections formed inside on the rear side of one of two half-split shape parts respectively for the upper side and the lower side, which incline the sheath on the storing position so that the top end of the injection needle abuts the inside on the front side of the protector and the top end of the injection needle as well as the sheath is supported inside the protector. Accordingly, the top end of the injection needle is prevented from moving in all directions without fail on the storing position, thereby enhancing safety of the medical needle device.

According to the medical needle device according to the tenth aspect of the present invention, the external shape of the sheath is sectorial or inverted trapezoidal in cross section, tightening the lower part thereof to narrow gradually from the upper part; and an internal shape of a part of the half-split shape part for the upper side of the protector to be capped on the sheath is sectorial or inverted trapezoidal in cross section, corresponding to the cross sectional shape of the sheath. Such a shape of the protector further prevents the sheath from moving in the rotational direction.

It is to be noted that the Japanese examined Patent Publication No. H06-7861 does not disclose that the protector and the wings are separate parts, the front part and the rear part of the protector are closed, the fitting force between the protector and the sheath is high and the medical needle device can be safely disposad after use as the medical needle device of the present invention.

### BRIEF DESCRIPTION OF THE DRAWINGS

Fig. 1 is a perspective view of a medical needle device according to embodiment 1 of the present invention;
Fig. 2 is an exploded perspective view of the medical needle device of Fig. 1;
Fig. 3 is a perspective view showing a state that an injection needle of the medical needle device of Fig. 1 is stored;
Fig. 4 is an enlarged sectional view showing important parts of a sheath of the medical needle device of Fig. 1;
Fig. 5 is a perspective view of another medical needle device according to embodiment 1 of the present invention;
Fig. 6 is an exploded perspective view of the medical needle device of Fig. 5;
Fig. 7 is a perspective view of a medical needle device according to embodiment 2 of the present invention;
Fig. 8 is a perspective view showing a protector of the medical needle device of Fig. 7;
Fig. 9 is a perspective view showing a sheath of the medical needle device of Fig. 7;
Fig. 10 is a perspective view of a medical needle device according to embodiment 3 of the present invention;
Figs. 11 show a protector of the medical needle device according to embodiment 3 of the present invention, of which (a) is a front perspective view and (b) is a rear perspective view;
Fig. 12 is a perspective view showing a sheath of the medical needle device of Fig. 10;
Figs. 13 show a protector of a medical needle device according to embodiment 4 of the present invention, of which (a) is a front perspective view and (b) is a rear perspective view;
Fig. 14 is a plane view of a sheath of a medical needle device according to a prior art;
Fig. 15 is a lateral view showing the medical needle device of Fig. 14 in use;
Fig. 16 is a plan view showing the medical needle device of Fig. 14 after use;
Fig. 17 is a cross section showing the medical needle device according to another prior art;
Fig. 18 is a cross section showing the medical needle device of Fig. 17 of which an injection needle is protruded;
Fig. 19 is a cross section showing the medical needle device according to still another prior art;
Figs. 20 is a longitudinal section showing the medical needle device of Fig. 19;
Fig. 21 is a cross section showing the medical needle device of Fig. 19 of which an injection needle is protruded;
Fig. 22 is a lateral view showing a protector of the medical needle device of Fig. 19;
Fig. 23 is an enlarged sectional view showing important parts of another sheath of the medical needle device according to first embodiment of the present invention;
Figs. 24 show an example of providing covers with a protector of the medical needle device according to the first embodiment of the present invention of which (a) is a perspective view and (b) is an exploded perspective view.
Figs. 25 are lateral views showing a state that the injection needle of the medical needle device according to the first embodiment of the present invention is stored, of which (a) shows a protector without covers and (b) shows a protector with covers;
Figs. 26 are lateral views showing a state that the injection needle of another medical needle device according to the first embodiment of the present invention is stored, of which (a) shows a sheath and an injection needle inclined upward on a front side and (b) shows the sheath and the injection needle inclined downward on the front side; and
Figs. 27 are cross sections showing various combinations of the sheath and the upper side of half-split shape part of the medical needle device according to the first embodiment of the present invention.

### BEST MODE FOR CARRYING OUT THE INVENTION

### (First Embodiment)

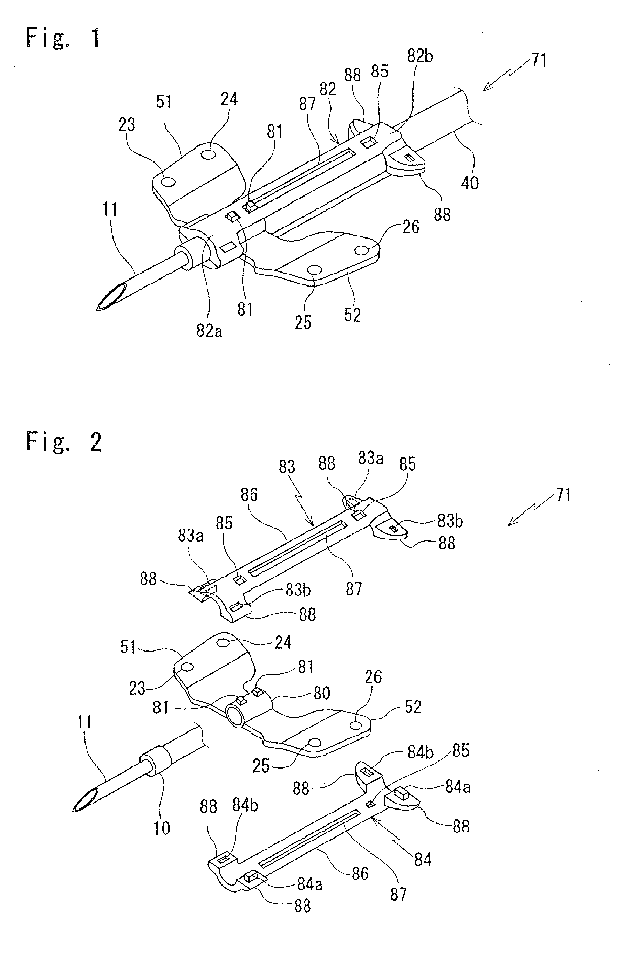

Referring to Figs. 1 to 4, a medical needle device71 according to the first embodiment will be described.

The medical needle device71 comprises a hub 10, a sheath 80 and a protector 82, which are separate parts.

The hub 10 is made of PC (polycarbonate) and has an injection needle 11 fixed at the top end thereof and a tube 40 capped with the rear end thereof.

The injection needle 11 is made of SUS (stainless steel) and the tube 40 is made of PVC (polyvinyl chloride).

The sheath 80 is a substantially cylindrical part which is made of PE (polyethylene), fit on the hub 10, has a pair of wings 51, 52 mounted on both right and left side surfaces thereof and supports the hub 10 in an interior side thereof.

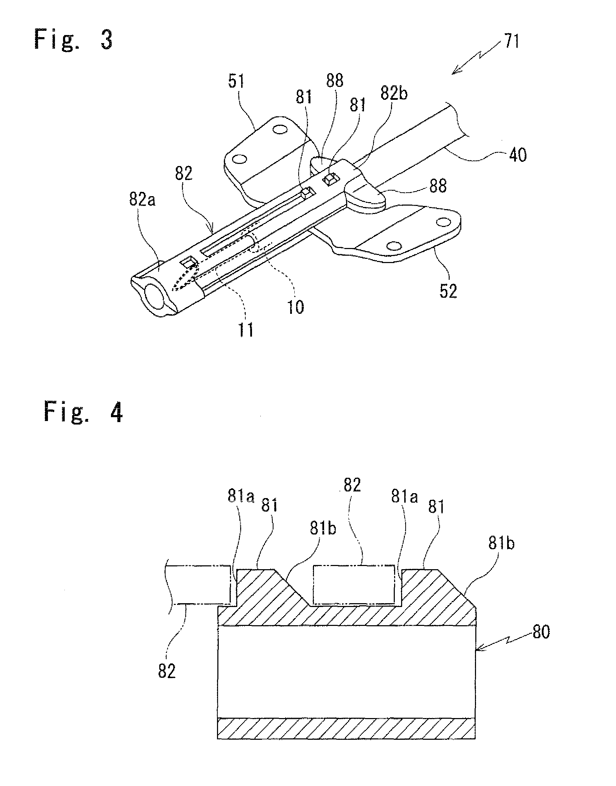

As shown in Fig. 4, the sheath 80 has two protrusions 81 respectively formed on a front side and a rear side of an upper part of an outer peripheral surface thereof. Rear parts 81b of both of the protrusions 81 are tapered, of which projecting amount on the outer peripheral surface of the sheath 80 increases gradually from the rear side toward front side. Front parts 81a of both of the protrusions 81 are wall surfaces roughly perpendicular to the upper part of an outer peripheral surface of the sheath 80. The protrusions 81 are high enough to have resistance necessary to be released from fitting state after fitting into anchoring holes 85 explained later.

The wings 51, 52 have wing convexes 23, 26 and wing concaves 24, 25 formed thereon and when the wings 51 and 52 are overlapped, the wing convex 23 engages with the wing concave 25 whereas the wing convex 26 engages with the wing concave 24.

The protector 82 is made of material harder than the sheath 80, of which an example is PC (polycarbonate) harder than a top end of the injection needle 11. The protector 82 comprises two half-split shape parts 83, 84 for upper and lower sides which have the same shape.

The two half-split shape parts 83, 84 fasten and cover the sheath 80 from the upper side and the lower side and have a space formed therebetween to enable slid of the sheath while a pair of the wings 51, 52 mounted on the sheath are protruded from the space. The sheath 80 together with the hub 10 is slidable back and forth relative to the protector 82 from a puncturing position shown in Fig. 1 where the injection needle 11 is protruded from the protector 82 to a storing position shown in Fig. 3 where the injection needle 11 is stored inside the protector 82.

The protector 82 formed of combination of the two half-split shape parts 83, 84 comprise a circular front part 82a which the injection needle 11 goes through; a circular rear part 82b which the tube 40 goes through; and connecting parts 86 connecting the front part 82a and the rear part 82b while forming spaces where a pair of the wings 51, 52 mounted on the sheath are protruded to enable the slid of the sheath. The connecting parts 86 extend from the front part 82a to the rear part 82b of the protector 82, connect upper parts on the front part 82a and the rear part 82b and lower parts on the front part 82a and the rear part 82b and have gaps formed between the right sides of the upper and lower parts and between the left sides of the upper and lower parts of the protector 82, which the wings 51, 52 can go through.

The protector 82 has anchoring holes 85 formed on the front side and the rear side of the upper part thereof. The anchoring hole 85 is a size that fits with one protrusion 81.

The protrusions 81 and the anchoring holes 85 function as a position controlling mechanism for positioning and fixing the sheath 80 relative to the protector 82 on the puncturing position where the injection needle 11 is protruded from the protector 82 and the storing position where the injection needle 11 is stored in the protector 82, The protrusion 81 formed on the front side of the upper part of the outer peripheral surface of the sheath 80 fits with the anchoring hole 85 formed on the front side of the upper part of the protector 82 on the puncturing position whereas the protrusion 81 formed on the rear side of the upper part of the outer peripheral surface of the sheath 80 fits with the anchoring hole 85 formed on the rear side of the upper part of the protector 82 on the storing position.

The upper side of the half-split shape part 83 of the protector 82 has the guide ditch 87 formed between the two anchoring holes 85 on the front side and the rear side thereof for guiding the protrusions 81 back and forth at the time of the slide of the sheath 80. The guide ditch 87 is roughly as wide as the protrusions 81 so as to prevent sheath 80 from rotating relative to an axis in which the protector 82 extends. In addition, an interval between the anchoring hole 85 on the front side and the guide ditch 87 and an interval between the anchoring hole 85 on the rear side and the guide ditch 87 are roughly the same as an interval between the two protrusions 81 respectively formed on the front side and the rear side of the sheath 80. Accordingly, when one protrusion 81 fits with the anchoring hole 85, the other protrusion 81 is inside the guide ditch 87.

Since the two half-split shape parts 83, 84 of the protector 82 have the same shapes, the lower side of the protector 82 where the protrusions 81 are not formed on the sheath 80 also has the anchoring holes 85 and the guide ditch 87 formed thereon. The anchoring holes 85 and the guide ditch 87 on the lower side of the protector 82 do not affect strength of the protector 82.

The upper side of the half-split shape part 83 has insertion convexes 83a, 83a respectively formed on a lower part on the front part and a lower part on the rear part on the right side thereof (the insertion convex 83a on the lower part on the front part extends in the front and rear direction whereas the insertion convex 83a on the lower part on the rear part extends in the right and left direction) and the convexes 83a, 83a are fit in insertion concaves 84b, 84b respectively formed on a front part and a rear part on the right side of the lower side of the half-split shape part 84. In the same way, the lower side of the half-split shape part 84 has insertion convexes 84a, 84a respectively formed on an upper part on the front part and an upper part on the rear part on the left side thereof (the insertion convex 84a on the upper part on the front part extends in the front and rear direction whereas the insertion convex 84a on the upper part on the rear part extends in the right and left direction) and the convexes 84a, 84a are fit in insertion concaves 83b, 83b respectively formed on a front part and a rear part on the left side of the upper side of the half-split shape part 83.

The protector 82 has supporting parts 88 formed on both side surfaces of a rear part 82b thereof, which protrude in such a manner as to project to the right and left so that doctors or the like can easily hold the protector 82 when sliding the sheath 80 relative to the protector 82.

In the course of puncturing upon binding the wings 51, 52, the bent wings 51, 52 apply binding force on the sheath 80 on which the wings are mounted relative to rotation in a circumferential direction but do not apply the binding force on the injection needle 11, the hub 10 and the tube 40. Such a structure enables to freely change direction of edge of the injection needle 11 to secure sufficient bloodstream.

The following will explain how to use and how to dispose the above-structured medical needle device 71.

Normally, when the medical needle devices 71 are provided to hospitals, a front-side protrusion 81 formed on an upper surface of the sheath 80 is fit in the anchoring hole 85 formed on the front part 82a of the protector 82 and the sheath 80 is on the puncturing position where the injection needle 11 is protruded as shown in Fig. 1. The injection needle 11 is capped with a cap (not shown) because an exposed injection needle 11 is dangerous.

Next, when using the medical needle device 71, the cap is removed from the injection needle 11 and a pair of the wings 51, 52 are overlapped and pinched. Then, the injection needle 11 is set on a blood vessel of a patient and then the bloodstream is secured. In case sufficient bloodstream is not secured, the injection needle 11 is rotated to change the direction of edge.

While sufficient bloodstream is secured, a pair of the wings 51, 52 are spread apart so as to come into contact with the patient's skin and are fastened on the patient's skin with adhesive tape (not shown).

After medical treatment, the injection needle 11 is removed from the patient's skin.

When disposing the medical needle device, the sheath 80 is slid backward relative to the protector 82 so as to fit a rear-side protrusion 81 formed on an upper surface of the sheath 80 in the anchoring hole 85 formed on the rear part 82b of the protector 82 as shown in Fig. 3, thereby storing the injection needle 11 inside the protector 82 on the storing position.

When storing the injection needle 11, the rear-side protrusion 81 formed on the upper surface of the sheath 80 is slid within the guide ditch 87 formed on the connecting parts 86 on the upper side of the half-split shape part 83 of the protector 82, thereby sliding the sheath 80 backward relative to the protector 82. Accordingly, the injection needle 11 is pulled straight backward and stored inside the protector 82.

In addition, since the rear part 82b of the protector 82 is circular and closed, the sheath 80 does not come off the protector 82 even in case an excessive force is applied on the sheath 80 for sliding backward relative to the protector 82. In case unreasonable force is applied so that the injection needle 11 slants and abuts the protector 82, the top end of the injection needle 11 does not break through a side surface of the protector 82 because the protector 82 is made of harder material than the sheath 80.

The position controlling mechanism of the above-structured medical needle device 71 for positioning and fixing the sheath 80 relative to the protector 82 on the puncturing position where the injection needle 11 is protruded from the protector 82 and the storing position where the injection needle 11 is stored inside the protector 82 controls movement of the injection needle 11 in the back and forth direction at the time of use and disposal of the medical needle device 71. Especially, at the time of the disposal, the position controlling mechanism prevents protrusion of the injection needle 11 fixed on the storing position outside the protector 82, thereby enhancing safety.

In addition, since the front part 82a and the rear part 82b of the protector 82 covering the sheath 80 are circular and closed, even in case an excessive force is applied on the sheath 80 when sliding the sheath 80 from the puncturing position to the storing position, the wings 51, 52 bump against the rear part 82b so that the sheath 80 does not come off the protector 82. Further, as compared with a prior art (see Figs. 19 to 22) where the rear part 82b of the protector 82 is partially open, deformation quantity of the rear part 82b is restricted, thereby enhancing fitting force of the sheath 80 positioned and fixed on the storing position.

As a result, it is unlikely that the injection needle 11 exposes so that the medical needle device 71 can be safely disposed.

In addition, since the protector 82 is made of harder material than the sheath 80, even in case the top end of the injection needle 11 abuts the side surface of the protector 82 on the storing position or while sliding the sheath 80 from the puncturing position to the storing position, the injection needle 11 does not break through the protector 82.

Further, since the protector 82 comprises two half-split shape parts 83, 84 for the upper side and the lower side, formation of so-called undercut on molds is prevented while the protector 82 is molded, thereby simplifying the molding

Especially, in the present embodiment, the two half-split shape parts 83, 84 for the upper side and the lower side have the same shapes which necessitate only one type of molds. Accordingly, the medical needle device 71 is cost effective and keeping parts of the medical needle device 71 before assembly becomes easier.

In addition, since the position controlling mechanism comprises the two protrusions 81 formed on the front side and the rear side of the upper part of the outer peripheral surface of the sheath 80 and the anchoring holes 85 formed on the front side and the rear side of the protector 82, which fit with the protrusions 81, the sheath 80 is positioned and fixed relative to the protector 82 without fail on the puncturing position and the storing position. Accordingly, the structure prevents the injection needle 11 from going into or protruding from the protector 82 unexpectedly, thereby enhancing safety of the medical needle device.

Further, when sliding the sheath 80, the protrusions 81 are moved from the anchoring hole 85 to the guide ditch 87 and from the guide ditch 87 to the anchoring hole 85 for position controlling. Accordingly, progress in each stage of operation can be recognized by sense of touch of the protrusions 81 with the protector 82. As a result, fitting state between the sheath 80 and the protector 82 can be recognized tactually as well as visually as in the conventional manner, thereby enhancing safety.

Formation of the guide ditch 87 for guiding the protrusions 81 back and forth at the time of the slide as well as tapered rear parts 81b of the protrusions 81, of which projecting amount on the outer peripheral surface of the sheath 80 gradually increases from the rear side toward front side improve operability at the time of the slide. More specifically, when the protrusions 81 are moved from the anchoring hole 85 on the front side to the guide ditch 87 and from the guide ditch 87 to the anchoring hole 85 on the rear side, the tapered rear parts 81b of the protrusions 81 easily climb over a part between the guide ditch 87 and the anchoring hole 85 as shown in Fig. 4 and nothing bumps against the protrusions 81 while the protrusions 81 are inside the guide ditch 87, thereby enhancing operability at the time of the slide.

Since front parts 81a of the protrusions 81 on the sheath 80 are wall surfaces roughly perpendicular to the upper part of the outer peripheral surface of the sheath 80, once the sheath 80 is fixed on the storing position, the sheath 80 is prevented from moving forward (left side direction in Fig. 4) relative to the protector 82 so that the injection needle 11 does not protrude from the front part.

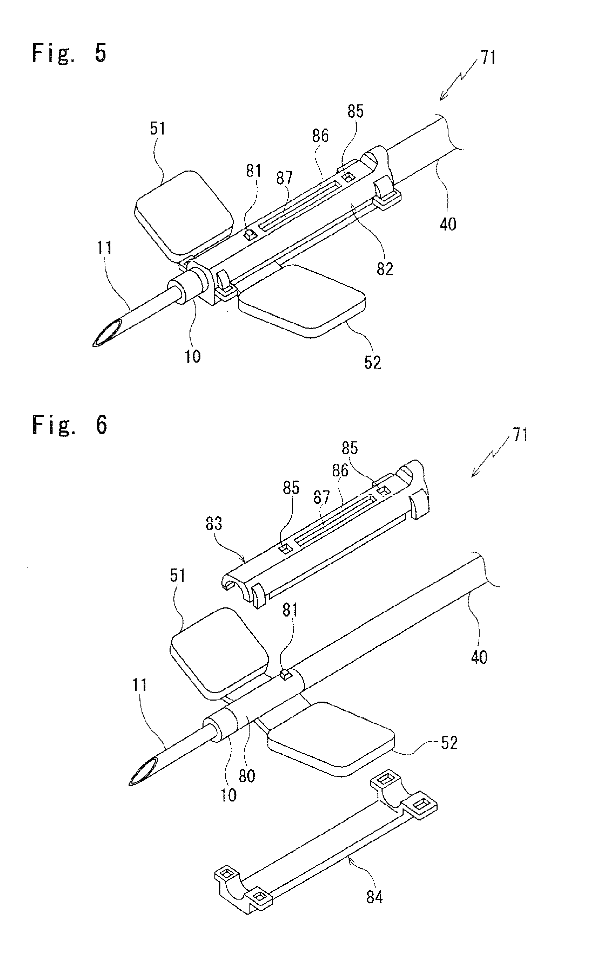

While the half-split shape parts 83, 84 for the upper part and the lower part of the present embodiment have the same shapes, the half-split shape parts 83, 84 for the upper part and the lower part do not necessarily have to have the same shapes as shown in Figs. 5 and 6 and the sheath 80 may have only one protrusion 81 formed on the outer peripheral surface thereof.

Width of spaces between the two half-split shape parts 83, 84 may be roughly the same as thickness of connecting parts of the wings 51, 52 relative to the sheath 80, where a pair of the wings 51, 52 are protruded to the right and left, so as to enable the movement of the sheath 80. Such a structure further prevents rotation of the sheath 80 for the effect of interaction between the wings 51, 52 and both of the half-split shape parts 83, 84 as well as interactions between the protrusions 81 and the anchoring holes 85 and between the protrusions 81 and the guide ditch 87, which prevent the rotation of the sheath 80 relative to the protector 82 (axial rotation of an axis extending in a longitudinal direction of the protector 82).

### (Second Embodiment)

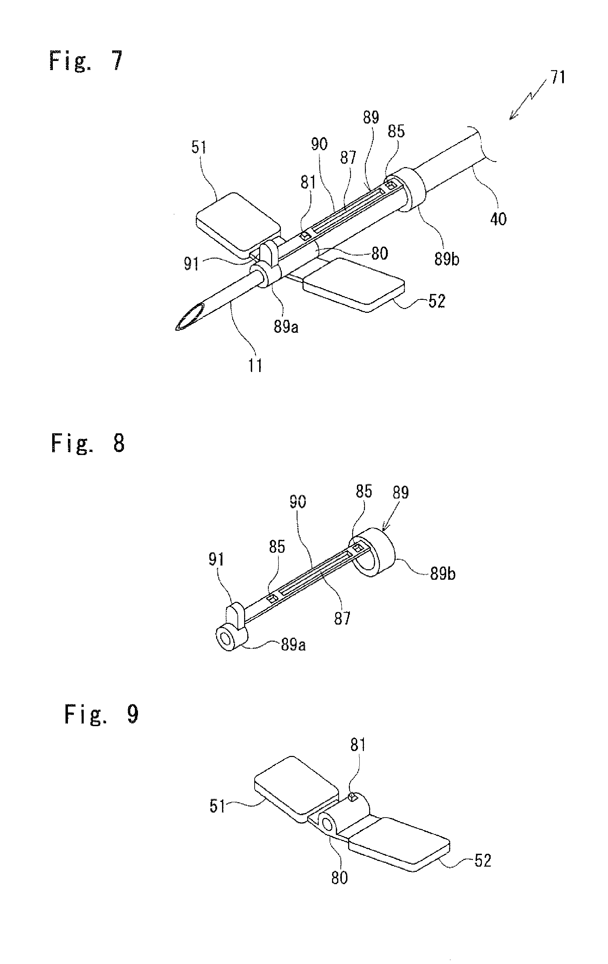

Referring to Figs. 7 to 9, the medical needle device71 according to the second embodiment will be described. When the second embodiment includes the same parts as those in the first embodiment, the same symbols are used.

Difference between the present embodiment and the first embodiment is the shape of a protector 89. Other parts of the present embodiment are the same as the first embodiment and perform the same effects as those of the first embodiment.

As shown in Fig. 8, connecting parts 90 of the protector 89 only connect an upper part of a circular front part 89a and an upper part of a circular rear part 89b. Therefore, the protector 89 except for the connecting parts 90, both side surfaces and lower part in another word, is a space. A pair of the wings 51, 52 are protruded from the space (more specifically, from the both side surfaces) so as to slide the sheath 80 relative to the protector 89.

The protector 89 has a supporting part 91 which protrudes upward formed on an upper part of the circular front part 89a thereof for supporting the protector 89 when sliding the sheath 80 relative to the protector 89. An inside diameter of the front part 89a is slightly larger than an outside diameter of the injection needle 11 so that the injection needle 11 goes through the front part 89a whereas an inside diameter of the rear part 89b is slightly larger than an outside diameter of the tube 40 so that the tube 40 goes through the rear part 89b.

Position controlling mechanism comprises one protrusion 81 shown in Fig. 9, which is formed on the upper part of the outer peripheral surface of the sheath 80 and anchoring holes 85 shown in Fig. 8, which are formed on a front side and a rear side of the connecting parts 90 of the protector 89, which fit with the protrusions 81.

The protector 89 comprises the guide ditch 87 formed between the anchoring holes 85 on the front side and rear side of the connecting parts 90 for guiding the protrusion 81 back and forth at the time of the slide of the sheath 80 relative to the protector 89.

According to the medical needle device 71 of the present embodiment, the connecting parts 90 of the protector 89 connect only the upper part of the front part 89a and the upper part of the rear part 89b and have the anchoring holes 85 respectively formed on the front side and the rear side thereof. Accordingly, although the protector 89 comprises a single part, the protector 93 does not cause undercut and is relatively easily molded.

In addition, the protector 89 which comprises the single part is cost effective.

### (Third embodiment)

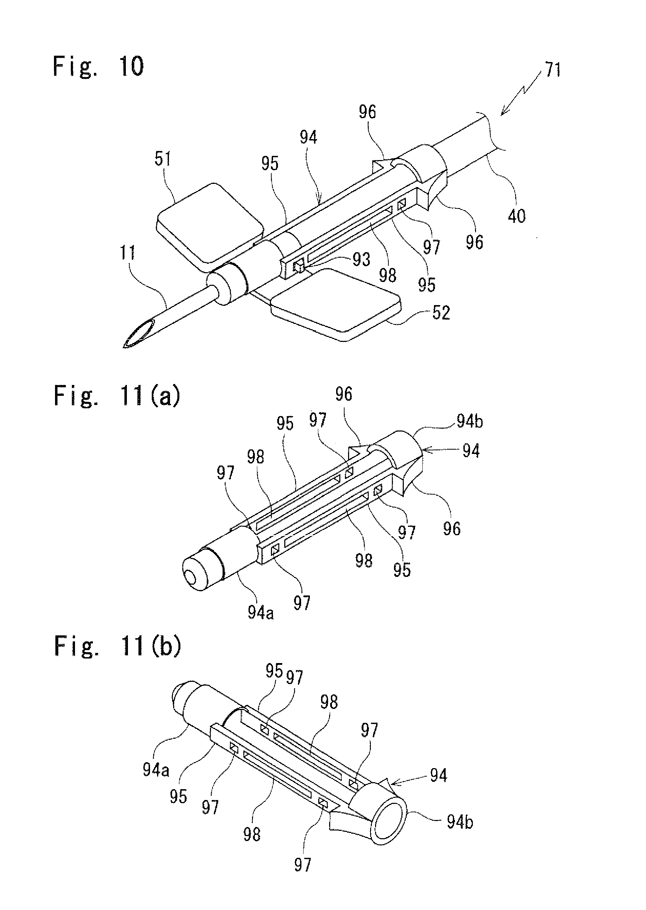

Referring to Figs. 10 to 12, the medical needle device71 according to the third embodiment will be described. When the third embodiment includes the same parts as those of the first embodiment, the same symbols are used.

Differences between the present embodiment and the first embodiment are position of protrusions 93 on a sheath 92 and the shape of a protector 94. Other parts of the present embodiment are the same as the first embodiment and perform the same effects as those of the first embodiment.

As shown in Fig. 11, connecting parts 95 of the protector 94 comprise two parts that connect both the right and left side surfaces of a circular front part 94a and both the right and left side surfaces of a circular rear part 94b. Therefore, the protector 94 except for the connecting parts 95, an upper part and a lower part of the protector 94 in another word, is a space. A pair of the wings 51, 52 are protruded from the space on a lower part of the connecting parts 95. In the course of assembly of the medical needle device71 of the present embodiment, the sheath 92 is fit in the protector 94 from a lower side of the protector 94 so that the connecting parts 95 of the protector 94 are mounted on both of the wings 51, 52.

The protector 94 has supporting parts 96 formed on right and left side surfaces of the rear part 94b thereof, which protrude to the right and left so that doctors or the like can hold the protector 94 when sliding the sheath 92 relative to the protector 94.

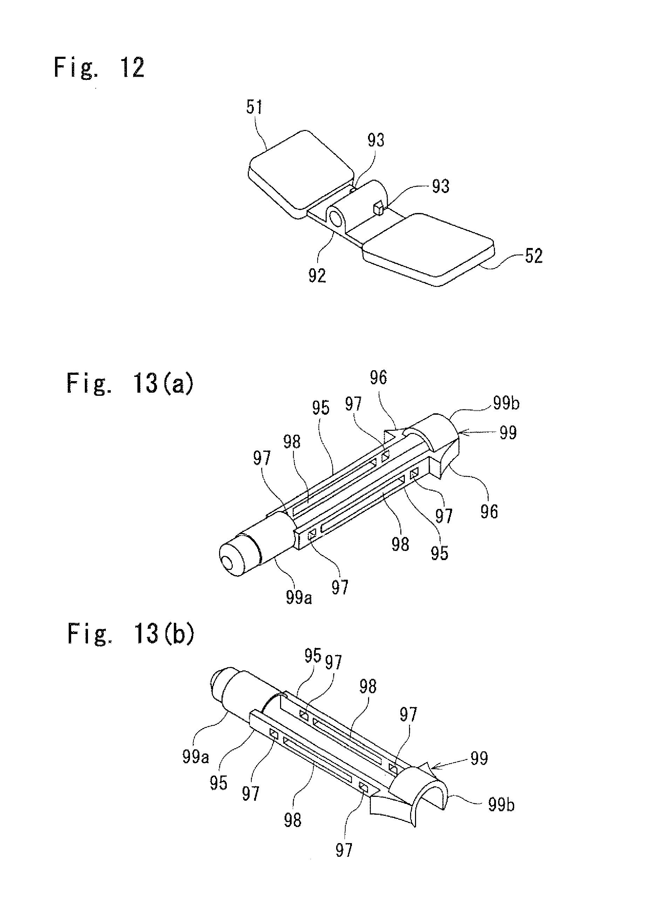

The position controlling mechanism comprises two protrusions 93 shown in Fig. 12, which are formed on the right and left side surfaces of the outer peripheral surface of the sheath 92 and anchoring holes 97 shown in Fig. 11, which are formed on the front side and the rear side of the two connecting parts 95 of the protector 94, which fit with the two protrusions 93.

The two connecting parts 95 respectively have the guide ditches 98 formed between the anchoring holes 97 on the front side and the rear side thereof for guiding the protrusions 93 back and forth when sliding the sheath 92 relative to the protector 89.

The medical needle device 71 of the present embodiment, having the connecting parts 95 of the protector 94 and the position controlling mechanism provided on two positions on the right and left side surfaces thereof performs high fitting force between the sheath 92 and the protector 94 so that once the sheath 92 is positioned and fixed on the storing position, the injection needle 11 hardly protrudes again, thereby enhancing safety.

In addition, the protector 94 which comprises the single part is simple in assembly and cost effective.

### (Fourth embodiment)

Referring to Figs. 10, 12 and 13, the medical needle device71 according to the fourth embodiment will be described. When the fourth embodiment includes the same parts as those in the first embodiment, the same symbols are used.

Difference between the present embodiment and the third embodiment is the shape of a protector. Other parts of the present embodiment are the same as the third embodiment and perform the same effects as those of the third embodiment.

As shown in Fig. 13, the protector 99 of the present embodiment comprises: a circular front part 99a which the injection needle 11 goes through; a half-split rear part 99b of which lower part is cut out for fitting the tube 40 therein; and two connecting parts 95 which connect both right and left side surfaces of the front part 99a and both right and left side surfaces of the rear part 99b.

In another word, the difference between the present embodiment and the third embodiment is that the protector 94 of the third embodiment comprises the circular rear part 99b whereas the protector 99 of the fourth embodiment comprises the half-split rear part 99b of which lower part is cut out.

According to the medical needle device 71 of the present embodiment, the half-split rear part 99b of the protector 99 of which the lower part is cut out enables assembly of the medical needle device 71 by the following procedure: protruding the injection needle 11 from the front part 99a of the protector 99 after fixing the injection needle 11 on the hub 10, fitting the sheath 92 on the hub 10, and connecting a tube 40 to a rear end 10b of the hub 10, and fitting a combined body comprising: the hub 10; the sheath 92; and the tube 40 with the protector 99 from the lower side. Accordingly, the assembly of the medical needle device 71 is simplified.

Position of the supporting parts 88, 91, 96 formed on the protector 82, 89, 94, 99 is not particularly limited to the positions shown in first to fourth embodiments and the supporting parts 88, 91, 96 can be formed on any position of the protector 82, 89, 94, 99 that enables the doctors or the like to easily hold the protector 82, 89, 94, 99. In addition, formation of the supporting parts 88, 91, 96 can be omitted as long as the slide is smoothly carried out.

While one or a plurality of the protrusion 81, 93 is formed on the sheath 80,92, the number of the protrusion 81, 93 is not limited as long as the sheath 80, 92 can be positioned and fixed relative to the protector 82, 89, 94, 99 on the puncturing position and the storing position and the slide is smoothly carried out.

While the rear parts 81b, 93b of the protrusions 81, 93 are tapered, shape of the rear parts 81b, 93b is not particularly limited and may also be roughly perpendicular to the sheath 80, 92 in the same manner as the front parts 81a, 93a. It is to be noted that the perpendicular rear parts 81b, 93b cause high resistance at the time of the slide, which requires preventive measures such as lowering height of the protrusions 81, 93.

In case two protrusions 81, 93 are formed on the sheath 80, 92, while the interval between the two protrusions 81, 93 is roughly the same as the interval between the anchoring hole 85, 97 on the front side and the guide ditch 87, 98 and the interval between the anchoring hole 85, 97 on the rear side and the guide ditch 87,98, any interval longer than the above-mentioned intervals is usable. In another word, any interval that allows one of the two protrusions 81, 93 to fit in the anchoring hole 85, 97 while the other protrusion 81,93 to be inside the guide ditch 87, 98 is usable.

While the sheath 80, 92 is made of PC, material of the sheath is not particularly limited and may also be made of PP (polypropylene) in order to improve moldability.

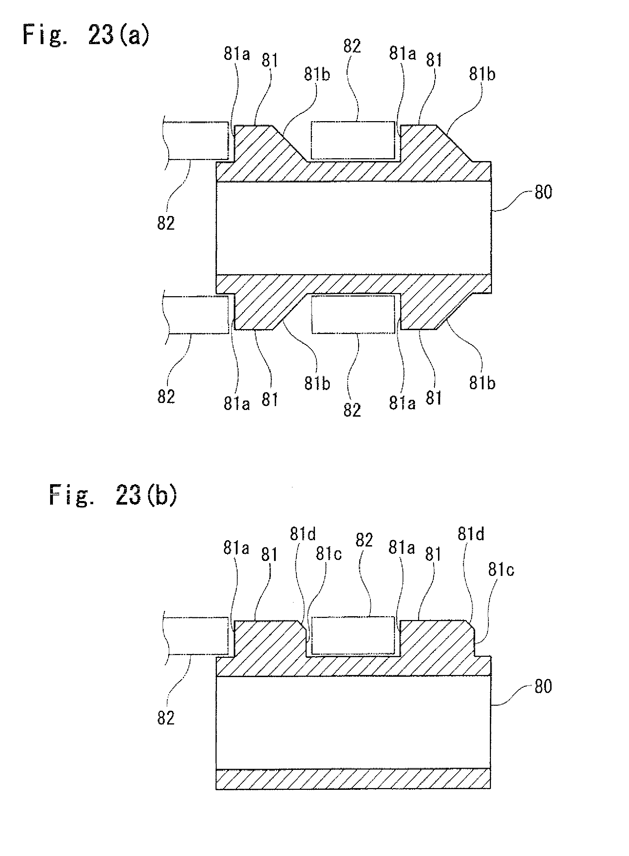

While the sheath 80 has two protrusions 81, 81 respectively formed on the front side and the rear side of the upper part of the outer peripheral surface thereof in the first embodiment, the sheath 80 may also have another two protrusions 81, 81 respectively formed on the front side and the rear side of the lower part of the outer peripheral surface thereof as shown in Fig. 23(a). The two protrusions 81, 81 formed on the lower part of the outer peripheral surface of the sheath 80 are also fit in the two anchoring holes 85 respectively formed on the front side and the rear side of the half-split shape part 84 for the lower side of the protector 82 on the puncturing position and the storing position (the front-side protrusion 81 is fit with the anchoring hole 85 formed on the front side of the half-split shape part 84 for the lower side on the puncturing position whereas the rear-side protrusion 81 is fit with the anchoring hole 85 formed on the rear side of the half-split shape part 84 for the lower side on the storing position). The protrusions 81 are guided through the guide ditch 87 at the time of the slide of the sheath 80.

Such a structure further improves the fitting force between the sheath 80 and the protector 82 on the puncturing position and the storing position because the sheath 80 and the protector 82 are fit with each other by two parts on the upper side and lower side. In addition, the protrusions 81 on the upper side and the lower side, guided through the two guide ditches 87 at the time of the slide of the sheath 80 further stabilize the slide.

While the rear part 81b of the protrusion 81 is linearly inclined from the outer peripheral surface of the sheath 80 toward the upper surface of the sheath 81 (Fig. 4) in the first embodiment, the rear part 81b of the protrusion 81 may comprise a vertical wall surface 81c vertically extending from the outer peripheral surface of the sheath 80 and an inclined surface 81d that linearly inclines from the vertical wall surface 81c toward the upper surface of the protrusion 81 so that the resultant protrusion 81 as a whole forms a pentagon as shown in Fig. 23 (b).

More specifically, the protrusion 81 according to the first embodiment (Fig. 4) may cause insufficient anchoring operation because it is difficult for operators to tactually recognize load softly applied when the protrusion 81 anchors to the anchoring hole 85 on the puncturing position and the storing position. On the other hand, the protrusion 81 shown in Fig. 23 (b) enables the operators to tactually recognize load properly applied when the protrusion 81 anchors to the anchoring hole 85 because the vertical wall surface 81c makes surface contact with the anchoring hole 85 and by applying additional operation force, the protrusion 81 climbs over the anchoring hole 85 with relatively less load for the effect of the inclined surface 81d so that the operators tactually recognize completion of the anchoring operation. As a result, the operators tactually recognize load fluctuation in each stage of the anchoring operation so that the operation can be performed easily and without fail.

While the protrusion 81(protrusion 81 on the right side in Fig. 4) on the rear side on the upper part of the outer peripheral surface of the sheath 80 is provided on the rear end of the sheath 80 in the first embodiment so that a flat part of the outer peripheral surface of the sheath 80 is not provided behind the rear part 81b, the protrusion 81 (protrusion 81 on the right side in Fig. 23 (a), Fig. 23 (b)) on the rear side on the upper part of the outer peripheral surface of the sheath 80 may be provided on a position slightly shifted forward from the rear end of the sheath 80 as shown in Fig. 23 (a), Fig. 23 (b) so that the flat part of the outer peripheral surface of the sheath 80 is provided behind the protrusion 81 on the rear side.

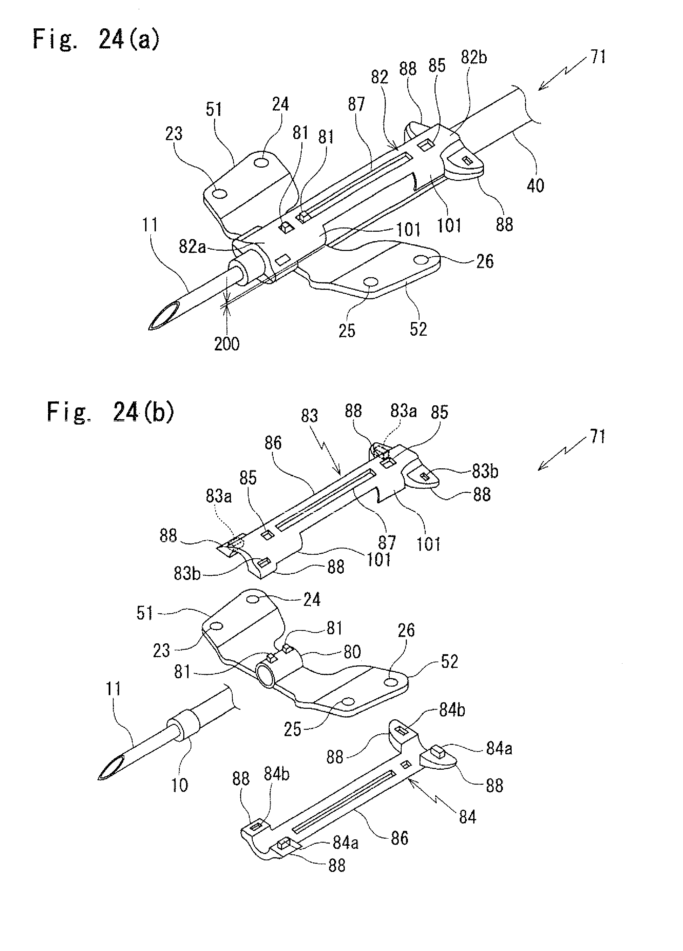

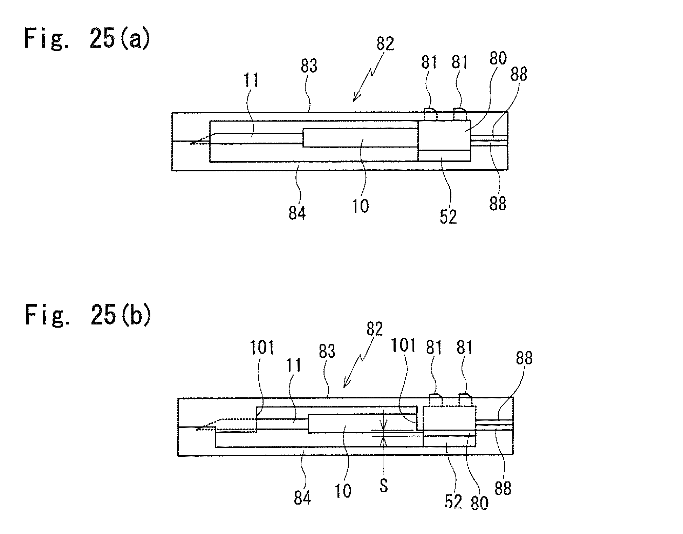

While a pair of the wings 51, 52 mounted on the sheath 80 are respectively protruded from the spaces formed between the two half-split shape parts 83, 84 for the upper side and the lower side of the protector 82 in the first embodiment, the protector 82 may include covers 101 for narrowing spaces 200 between upper parts of a pair of the wings 51, 52 and parts of the protector 82 facing the upper parts of a pair of the wings 51, 52 on the puncturing position and the storing position as shown in Fig. 24 (a), Fig. 24 (b) and Fig. 25 (b). In the Figs., four covers 101 are provided on the right and left on the front side and the right and left on the rear side of the protector 82 and distances between upper parts of a pair of the wings 51, 52 and lower ends of the covers 101 are the spaces 200. The covers 101 are semi-arcuate in cross-section along the outer peripheral surface of the protector 82, which do not protrude outward compared with the outer peripheral surface of the protector 82. Fig. 25 (a) shows a comparative embodiment where the protector 82 does not have the covers 101.

According to the structure, even in case strong force is applied on the sheathe 80 or the wings 51, 52 in the rotational direction, which is so strong as to release the fitting by positioning and fixing the protrusions 81 relative to the anchoring holes 85 upon fitting the protrusions 81 in the anchoring holes 85, the wings 51, 52 immediately come into contact with the covers 101 so that further rotation is prevented. The shorter the space between the upper parts of the wings 51, 52 and the covers 101 is within a range in which the sheath 80 is slidable, the smaller the movement in the rotational direction is.

In addition, formation of the covers 101 wide enough to hide the top end of the injection needle 11 leads to a sense of security to operators.

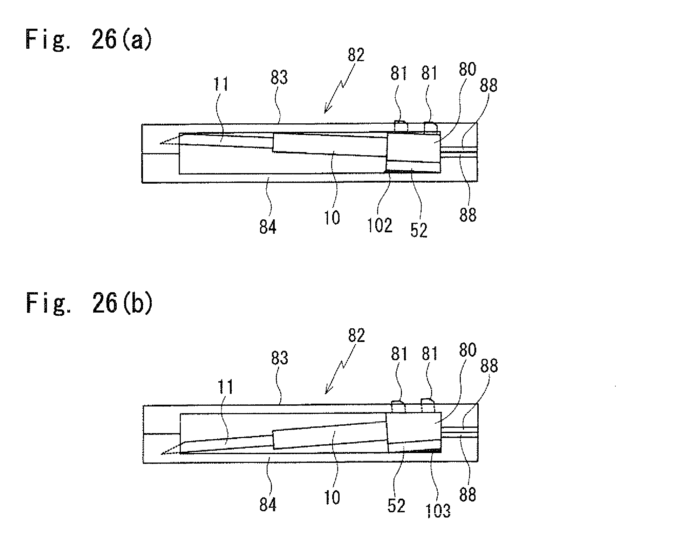

While the injection needle 11 is positioned in parallel with longitudinal direction in which the protector 82 extends in the storing position as shown in Fig. 25 (a) in the first embodiment, the injection needle 11 may be inclined inside the protector 82 so that the top end of the injection needle 11 abuts the inside on the front side of the protector 82 as shown in Fig. 26 (a) and Fig. 26 (b).

In Fig. 26 (a), the protector 82 has an inclined projection 102 formed inside on the rear side of the half-split shape part 84 for the lower side, which inclines the sheath 80 backward on the storing position by heightening a front part of the sheath 80 compared with a rear part so that the top end of the injection needle 11 abuts the inside on a front upper side of the protector 82. In Fig. 26 (b), the protector 82 has an inclined projection 103 formed inside on the rear side of the half-split shape part 84 for the lower side, which inclines the sheath 80 forward at the storing position by heightening the rear part of the sheath 80 compared with the front part so that the top end of the injection needle 11 abuts the inside on a front lower side of the protector 82. More specifically, the inclined projection 102 shown in Fig. 26 (a) has a linearly enlarged projecting amount on the front side compared with the rear side whereas the inclined projection 103 shown in Fig. 26 (b) has a linearly enlarged projecting amount on the rear side compared with the front side. The inclined projection 102 may be formed inside on the rear side of the half-split shape part 82 for the upper side of the protector 82 so that the top end of the injection needle 11 abuts the inside on the front lower side of the protector 82 or the inclined projection 103 may be formed inside on the rear side of the half-split shape part 82 for the upper side of the protector 82 so that the top end of the injection needle 11 abuts the inside on the front upper side of the protector 82.

According to the structure, since the top end of the injection needle 11 as well as the sheath 80 is supported inside the protector 82, the top end of the injection needle 11 is prevented from moving in all directions without fail on the storing position. Accordingly, even in case force in the right and left direction (lateral direction) is applied on the sheath 80 or the wings 51, 52 made of relatively soft material, the top end of the injection needle 11 is prevented from moving in accordance with the movement of the sheath 80 or the wings 51, 52.

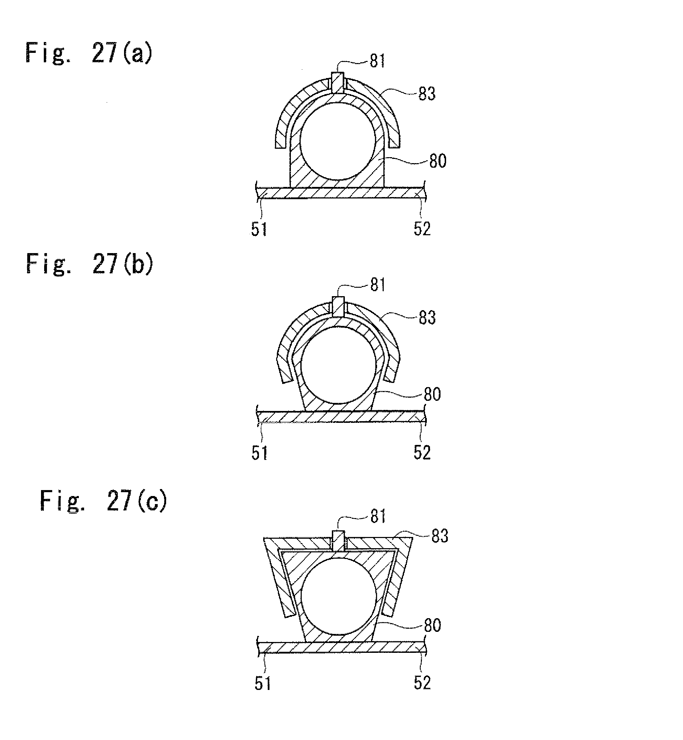

While an external shape of the sheath 80 is roughly circular in cross section and a part of the half-split shape part 83 for the upper side of the protector 82 to be capped on the sheath 80 is roughly semicircular in cross section, corresponding to the cross sectional shape of the sheath 80 in the first embodiment as shown in Fig. 27 (a), the external shape of the sheath 80 may be sectorial in cross section tightening the lower part thereof to narrow gradually from the upper part and the internal shape of a part of the half-split shape part 83 for the upper side of the protector 82 to be capped on the sheath 80 may be sectorial in cross section, corresponding to the cross sectional shape of the sheath 80 as shown in Fig. 27 (b) or the external shape of the sheath 80 may be inverted trapezoidal in cross section tightening the lower part thereof to narrow gradually from the upper part and the internal shape of a part of the half-split shape part 83 for the upper side of the protector 82 to be capped on the sheath 80 may be inverted trapezoidal in cross section, corresponding to the cross sectional shape of the sheath 80 as shown in Fig. 27 (c).

According to the structure, in case force is applied on the sheath 80 in the axial rotational direction of the sheath 80 in which the sheath 80 extends relative to the half-split shape part 83 for the upper side of the protector 82, both ends of the sectorial part or both corner ends on upper part of the inverted trapezoidal part of the sheath 80 engage with the half-split shape part 83 for the upper side so that the sheath 80 is prevented from moving in the rotational direction, thereby preventing unexpected rotation and enhancing safety of the medical needle device.

## Claims

1. A medical needle device comprising: a hub having an injection needle and a tube respectively fixed at a top end and a rear end thereof; a cylindrical sheath which is fit on the hub, having a pair of wings mounted on both right and left side surfaces thereof and supporting the hub in an interior side thereof; and a protector fit on the sheath, where said sheath together with the hub is slidable back and forth from a puncturing position where the injection needle is protruded to a storing position where the injection needle is stored, wherein:
said protector comprises: a circular front part which said injection needle goes through; a circular rear part which said tube goes through; and connecting parts connecting said front part and said rear part, which have spaces for protruding said pair of wings on said sheath therefrom for enabling slide of said sheath; said protector is made of harder material than that of said sheath: and
an outer peripheral surface of said sheath and a front side and a rear side of said protector form a position controlling mechanism for positioning and fixing said sheath relative to said protector on said puncturing position and said storing position.

2. The medical needle device as claimed in Claim 1, wherein:
said protector comprises two half-split shape parts for an upper side and a lower side, covers said sheath in such a manner as to fasten said sheath from the upper side and the lower side and has the space between said half-split shape parts for enablilng the slide of said sheath.

3. The medical needle device as claimed in Claim 2, wherein:
said position controlling mechanism comprises protrusions formed on an upper part of the outer peripheral surface of said sheath and anchoring holes formed on the front side and the rear side of said protector, which fit with said protrusions; and said half-split shape part for the upper side of said protector has a guide ditch formed between said anchoring holes on the front side and rear side thereof for guiding said protrusions back and forth at a time of said slide.

4. The medical needle device as claimed in Claim 2 or 3, wherein:
both of said half-split shape parts for the upper side and the lower side have same shapes.

5. The medical needle device as claimed in Claim 1, wherein:
said connecting parts of the protector only connect an upper part of said front part and an upper part of said rear part; and said position controlling mechanism comprises the protrusion formed on the upper part of the outer peripheral surface of said sheath and the anchoring holes formed on the front side and the rear side of the connecting parts of said protector, which fit with said protrusion; and said connecting parts have the guide ditch formed between said anchoring holes on the front side and the rear side thereof for guiding said protrusion back and forth at the time of said slide.

6. The medical needle device as claimed Claim 1, wherein:
said connecting parts of said protector comprise two parts that connect both right and left side surfaces of said front part and both right and left side surfaces of said rear part; said position controlling mechanism comprises two protrusions respectively formed on the right and left side surfaces of the outer peripheral surface of said sheath and the anchoring holes formed on the front side and the rear side of the two connecting parts of said protector, which fit with said two protrusions; and said two connecting parts respectively have the guide ditches formed between said anchoring holes on the front side and the rear side thereof for guiding said protrusions back and forth at the time of said slide.

7. A medical needle device comprising: a hub having an injection needle and a tube respectively fixed at a top end and a rear end thereof; a cylindrical sheath which is fit on the hub, having a pair of wings mounted on both right and left side surfaces thereof, and supporting the hub in an interior side thereof; and a protector fit on the sheath, where said sheath together with the hub is slidable back and forth from a puncturing position where the injection needle is protruded to a storing position where the injection needle is stored, wherein:
said protector comprises: a circular front part which said injection needle goes through; a half-split rear part of which lower part is cut out for fitting said tube therein; and two connecting parts which connect both right and left side surfaces of said front part and both right and left side surfaces of said rear part, where said pair of wings on said sheath protrude from lower parts of said connecting parts for enabling slid of said sheath; said protector is made of harder material than said sheath; and
two protrusions formed both on right and left side surfaces of an outer peripheral surface of said sheath and anchoring holes formed on the front side and the rear side of the two connecting parts of said protector, which fit with said protrusions form a position controlling mechanism for positioning and fixing said sheath relative to said protector on said puncturing position and said storing position; and said two connecting parts have guide ditches formed between said anchoring holes on the front side and the rear side thereof for guiding said protrusions back and forth at a time of said slide.

8. The medical needle device as claimed in any one of Claims 1 to 4, wherein:
said protector includes covers for narrowing spaces between upper parts of said pair of wings on said sheath and parts of said protector facing said upper parts of a pair of said wings on said puncturing position and said storing position.

9. The medical needle device as claimed in any one of Claims 2 to 4, wherein:
said protector has inclined projections formed inside on the rear side of one of two half-split shape parts for the upper side and the lower side, which incline said sheath (decrease or increase projecting amount gradually from a front side toward a rear side) on said storing position so that the top end of said injection needle abuts an inside on the front side of said protector.

10. The medical needle device as claimed in any one of Claims 2 to 4, wherein:
an external shape of said sheath is sectorial or inverted trapezoidal in cross section, tightening the lower part thereof to narrow gradually from the upper part; and an internal shape of a part of the half-split shape part for the upper side of said protector to be capped on said sheath is sectorial or inverted trapezoidal in cross section, corresponding to a cross sectional shape of said sheath.
